# EUROPEAN PATENT SPECIFICATION

(11) **EP 2 675 529 B1**
(45) Date of publication and mention of the grant of the patent: **11.04.2018**
(21) Application number: 11704521.1
(22) Date of filing: 15.02.2011
(51) Int. Cl.: A61Q 11/00, A61K 8/33, A61K 8/34, A61K 8/42, A61K 8/49, A61K 8/92, A61K 8/27

(54) **TASTE-MASKED ORAL CARE COMPOSITIONS**
GESCHMACKSMASKIERTE MUNDPFLEGEZUSAMMENSETZUNGEN
COMPOSTIONS DE SOIN BUCCAL À GOÛT MASQUÉ

(43) Date of publication of application: 25.12.2013
(73) Proprietor: Colgate-Palmolive Company, New York, NY 10022 (US)
(72) Inventor: VOGT, Robert, Princeton New Jersey 08550 (US); KOHRS, Karsten, Berkley Heights New Jersey 07922 (US); FISHER, Steven, Wade, Middlesex New Jersey 08846 (US); CAMPBELL, Thomas, Flemington New Jersey 08822 (US); PRENCIPE, Michael, West Windsor New Jersey 08550 (US)
(74) Representative: Wibbelmann, Jobst
(86) International application number: PCT/US2011/024874
(87) International publication number: WO 2012/112141

(56) References cited:
- WO-A1-00/28952
- WO-A1-98/11867
- WO-A2-2008/005548
- JP-A- 11 071 252
- JP-A- 2003 073 282
- JP-A- 2003 137 755
- US-B1- 6 306 372
- DATABASE WPI Week 201075 Thomson Scientific, London, GB; AN 2010-N34531 XP002662844, -& JP 2010 235557 A (SUNSTAR CHEM IND CO LTD) 21 October 2010 (2010-10-21)

## Description

### BACKGROUND

There is an ongoing need to improve the taste profile of oral care compositions containing metal salts.

### SUMMARY

The present invention provides a flavor component according to claim 1 for use in an oral care composition containing a metal salt. Preferred features are defined in the dependent claims.

The present invention also provides a composition according to claim 3 comprising a flavor component of the present invention. Preferred features are defined in the dependent claims.

Methods for preparing and using the flavor components and compositions described herein are also discussed.

### DETAILED DESCRIPTION

As used throughout, ranges are used as shorthand for describing each and every value that is within the range. Any value within the range can be selected as the terminus of the range.

In the event of a conflict in a definition in the present disclosure and that of a cited reference, the present disclosure controls.

Described herein is a flavor component for use in an oral care composition containing a metal salt, comprising: a taste-masking agent comprising: cinnamic aldehyde; eugenol; and eucalyptol; and one or more flavoring agents selected from: L-menthol; N-ethyl-para-menthan-3-carboxamide; anethole; peppermint oil; spearmint oil; corn mint oil; and a combination of two or more thereof.

The present invention provides a flavor component comprising:
from 30 to 50%, by weight, L-menthol; from 5 to 10%, by weight, N-ethyl-para-menthan-3-carboxamide; from 5 to 14%, by weight, anethole; from 0.1 to 1%, by weight, cinnamic aldehyde; from 0.1 to 1%, by weight, eugenol; from 1 to 5%, by weight, eucalyptol; from 20 to 40%, by weight, peppermint oil; from 1 to 5%, by weight, spearmint oil; and from 5 to 10%, by weight, corn mint oil.

In some embodiments the flavor component comprises: from 35 to 45%, by weight, L-menthol; from 5 to 8%, by weight, N-ethyl-para-menthan-3-carboxamide; from 8 to 12%, by weight, anethole; from 0.5 to 0.9%, by weight, cinnamic aldehyde; from 0.5 to 0.9%, by weight, eugenol; from 2 to 4%, by weight, eucalyptol; and from 22 to 27%, by weight, peppermint oil.

The present invention also provides an oral care composition comprising: any one of the flavor components of the present invention; one or more metal salts selected from: a zinc salt; a calcium salt; a copper salt; an iron salt; a magnesium salt; a manganese salt; and a combination of two or more thereof; and an orally acceptable carrier, wherein the total concentration of said one or more metal salts is from 0.01 to 5 % by weight of the composition.

In some embodiments, at least one of the one or more metal salts is a zinc salt selected from: zinc oxide; zinc sulfate; zinc chloride; zinc citrate; zinc lactate; zinc gluconate; zinc malate; zinc tartrate; zinc carbonate; zinc phosphate; and a combination of two or more thereof. In some embodiments, the zinc salt is selected from: zinc oxide; zinc citrate; zinc gluconate; zinc lactate; and a combination of two or more thereof. In other embodiments, the zinc salt is selected from: zinc oxide; zinc citrate; and a combination of two or more thereof. In some embodiments, the zinc salt is zinc citrate.

The metal salt is present at a concentration of from 0.01% to 5%, by weight of the composition. In some embodiments, the metal salt is present at a concentration of from about 0.1 to about 4%, by weight of the composition. While other embodiments provide compositions wherein the metal salt is present at a concentration of from 1 to 3%, by weight. In some embodiments, the metal salt is present at a concentration of about 2%, by weight of the composition.

In some embodiments, the flavor component is present at a concentration of from 0.5 to 2.5%, by weight. Some embodiments provide a composition wherein the flavor component is present at a concentration of from about 0.8 to about 1.5%, by weight. Other embodiments provide a composition wherein the flavor component is present at a concentration of about 1%, by weight.

In some embodiments, the compositions further comprise one or more components selected from: a fluoride ion source; a tartar control agent; a buffering agent; an abrasive; and a combination of two or more thereof. In some embodiments, at least one of the one or more components is a fluoride ion source selected from: stannous fluoride, sodium fluoride, potassium fluoride, sodium monofluorophosphate, sodium fluorosilicate, ammonium fluorosilicate, amine fluoride, ammonium fluoride, and a combination of two or more thereof.

The amount of the flavor component included in a metal salt containing composition is generally and functionally described as an amount effective to mask the taste of the metal salt. In some embodiments, the flavor component mitigates the negative attributes of the metal salts without detracting from consumer acceptance of the product.

Other optional additives may be included. Among such optional additives, included are those provided in order to change appearance or aesthetic appeal, and/or to preservative the final product, and/or for taste/cosmetic appeal and/or as therapeutic and prophylactic ingredients for oral health, prevention or treatment of a condition or disorder of hard or soft tissue of the oral cavity, or the prevention or treatment of a physiological disorder or condition.

Colorants such as dyes may be food color additives presently certified under the Food Drug & Cosmetic Act for use in food and ingested drugs, including dyes such as FD&C Red No. 3 (sodium salt of tetraiodofluorescein), Food Red 17, disodium salt of 6-hydroxy-5-{(2-methoxy-5-methyl-4-sulphophenyl)azo}-2-naphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sul- fophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-.DELTA.-3,5-cycl- ohexadienimine], FD&C Blue No. 1 (disodium salt of dibenzyldiethyl-diamino- triphenylcarbinol trisulfonic acid anhydrite), FD&C Blue No. 2 (sodium salt of disulfonic acid of indigotin) and mixtures thereof in various proportions. Typically, colorants if included are present in very small quantities.

Sweeteners include both natural and artificial sweeteners. Suitable sweetener include water soluble sweetening agents such as monosaccharides, disaccharides and polysaccharides such as xylose, ribose, glucose (dextrose), mannose, galactose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame). In general, the effective amount of sweetener is utilized to provide the level of sweetness desired for a particular composition, will vary with the sweetener selected. This amount will normally be about 0.001% to about 5% by weight of the composition. In some embodiments, the sweetener is sodium saccharin and present at about 0.01% by weight of the composition.

Optional breath freshening agents may be provided. Any orally acceptable breath freshening agent can be used, including without limitation zinc salts such as zinc gluconate, zinc citrate and zinc chlorite, alpha-ionone and mixtures thereof. One or more breath freshening agents are optionally present in a breath freshening effective total amount.

Optionally, the composition may include a tartar control (anticalculus) agent. Tartar control agents among those useful herein include phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof, wherein sodium can optionally be replaced by potassium or ammonium. Other useful anticalculus agents include polycarboxylate polymers and polyvinyl methyl ether/maleic anhydride (PVME/MA) copolymers, such as those available under the Gantrez™ brand from ISP, Wayne, N.J.

In some embodiments, tartar control agent is present at a concentration of from about 0.01 to 10%, by weight. In some embodiments, the tartar control agent is present at a concentration of about 1%, by weight. In some embodiments, sodium phosphate monobasic is present at a concentration of from about 0.01 to about 5%, by weight. In some embodiments, sodium phosphate monobasic is present at a concentration of about 1%, by weight. In some embodiments, disodium phosphate is present at a concentration of from about 0.01 to about 5%, by weight. In some embodiments, disodium phosphate is present at a concentration of about 0.15%, by weight.

Other optional additives include antimicrobial (e.g., antibacterial) agents. Any orally acceptable antimicrobial agent can be used, including triclosan (5-chloro-2-(2,4-dichlorophenoxy)phenol); zinc and stannous ion sources; quaternary ammonium compounds such as cetylpyridinium chloride (CPC); bisguanides such as chlorhexidine; and benzalkonium chloride. A further illustrative list of useful antibacterial agents is provided in U.S. Pat. No. 5,776,435 to Gaffar, et al.

Antioxidants are another class of optional additives. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

Also optional, a saliva stimulating agent, useful for example in amelioration of dry mouth may be included. Any orally acceptable saliva stimulating agent can be used, including without limitation food acids such as citric, lactic, malic, succinic, ascorbic, adipic, fumaric, and tartaric acids, and mixtures thereof. One or more saliva stimulating agents are optionally present in a saliva stimulating effective total amount.

Optionally, an antiplaque (e.g., plaque disrupting) agent may be included. Any orally acceptable antiplaque agent can be used, including without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and mixtures thereof.

Optional desensitizing agents include potassium citrate, potassium chloride, potassium tartrate, potassium bicarbonate, potassium oxalate, potassium nitrate, strontium salts, and mixtures thereof.

Optionally, an inorganic or a natural or synthetic thickener or gelling agent may be present. In some embodiments, the thickener or gelling agent is present in the amount of from about 0.10 to about 5% by weight, or from about 0.2 to about 1% by weight. Suitable thickeners or gelling agents useful in the practice of the present invention include, for example and not limitation, inorganic thickening silicas such as amorphous silicas available from Huber Corporation under the trade designation Zeodent 165, Irish moss, iota-carrageenan, gum tragacanth, and polyvinylpyrrolidone.

In some embodiments, the compositions include a dental abrasive or combination of dental abrasive agents known in the art. Abrasives suitable for use in the compositions of the present invention include, but are not limited to, silica, calcined alumina, sodium bicarbonate, calcium carbonate, dicalcium phosphate and calcium pyrophosphate. If included, the abrasive is generally present at a concentration of from about 3 to about 50% by weight.

In some embodiments, surfactants are used in the compositions of the present invention. Suitable examples of surfactants include water-soluble salts of higher fatty acid monoglyceride monosulfates, such as the sodium salt of the monsulfated monoglyceride of hydrogenated coconut oil fatty acids, cocamidopropyl betaine, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine.

If included, surfactants are generally present at a concentration of from about 0.1 to about 5%, by weight of the composition. In some embodiments, the surfactant is present at a concentration of from about 0.5 to about 4%, by weight of the composition. In some embodiments, the surfactant is present at a concentration of from about 1 to about 3%, by weight of the composition. In some embodiments, the surfactant is present at a concentration of about 2%, by weight of the composition

For illustrative purposes, compositions of the present invention can be made by combining water, humectants, e.g. glycerin, sorbitol, polyethylene glycol in a conventional mixer until the mixture becomes a homogeneous gel phase. Into the gel phase are added the metal salt and, if included, abrasive(s) and the like. These ingredients are mixed until a homogeneous phase is obtained. Thereafter, a flavor component as described herein along with any thickeners, colorants, and surfactant are added and the ingredients are mixed at high speed in vacuum of about 20 to 100 mmHg.

Embodiments of the present invention are further described in the following examples. The examples are merely illustrative and do not in any way limit the scope of the invention as described and claimed.

### EXAMPLES

### Example 1

Flavor components according to the present invention (6 and 11) are shown below in Table 1, with amounts of components in weight %. Components 1-5 and 7-10 are not according to the present invention. These flavor components can be made via conventional methods.

**Table 1**

| | **Flavor Component** | | | | | | | | | | |
|---|---|---|---|---|---|---|---|---|---|---|---|
| | 1 | 2 | 3 | 4 | 5 | 6 | 7 | 8 | 9 | 10 | 11 |
| **Ingredient** | **% w/w** | | | | | | | | | | |
| L-menthol | 35 | 47 | 41 | -- | 38 | 39 | 42 | -- | -- | 36 | 43 |
| N-ethyl-para-menthan-3-carboxamide | -- | 5.4 | -- | 7.6 | -- | 5.5 | 7.2 | 6.1 | -- | 5.7 | 7.3 |
| Anethole | 8.3 | 11.2 | -- | -- | 8.7 | 10 | 9.6 | -- | -- | 9.3 | 8.5 |
| Cinnamic aldehyde | 1.4 | 0.6 | 0.8 | 0.5 | 0.3 | 0.7 | 0.5 | 0.8 | 0.6 | 0.9 | 0.8 |
| Eugenol | 0.2 | 0.8 | 0.8 | 0.9 | 1.6 | 0.7 | 0.6 | 0.9 | 0.6 | 0.8 | 0.5 |
| Eucalyptol | 1.1 | 2.6 | 2.3 | 3.1 | 5.2 | 3.6 | 2.4 | 3 | 2.7 | 3.2 | 2.8 |
| Peppermint oil | 24.8 | -- | -- | 23.7 | -- | 25 | 22.6 | -- | -- | 23 | 26.1 |
| Spearmint oil | -- | 4.2 | -- | -- | 3 | 2 | -- | 1.8 | -- | 4.1 | 1.2 |
| Cornmint oil | 6.2 | -- | -- | 7.2 | -- | 5 | -- | 5.4 | 6.3 | 7.6 | -- |

### Example 2

The formulations of an exemplary composition of the present invention (Composition I) and two comparative examples (Compositions X and Y) are described in Table 2 (below).

**Table 2**

| | **Composition** | | |
|---|---|---|---|
| | **I** | **X** | **Y** |
| **Ingredient** | % **w/w** | | |
| Water | 17.8 | 17.8 | 15.3 |
| Sodium saccharin | 0.3 | 0.3 | 0.3 |
| Sodium monofluorophosphate | 1.1 | 1.1 | -- |
| Sodium fluoride | -- | -- | 0.24 |
| Triclosan | -- | -- | 0.3 |
| Zinc citrate trihydrate | 2 | 2 | -- |
| Glycerin | 16.5 | 16.5 | 20 |
| PEG 600 | 3 | 3 | -- |
| Propylene glycol | -- | -- | 0.5 |
| Sodium CMC | 0.6 | 0.6 | 1.1 |
| Carrageenan | -- | -- | 0.5 |
| Xanthan gum | 0.4 | 0.4 | -- |
| Sorbitol (70% solution) | 17.8 | 17.8 | 20.9 |
| Tetrapotassium pyrophosphate | 2.4 | 2.4 | -- |
| PVM/MA Co-polymer | 11.5 | 11.5 | 15 |
| Sodium hydroxide (50% solution) | 1.3 | 1.3 | 1.2 |
| Titanium dioxide | 1 | 1 | 0.75 |
| Abrasive silica | 20 | 20 | 18.5 |
| Thickening silica | 1.8 | 1.8 | 3 |
| Sodium lauryl sulfate | 1.5 | 1.5 | 1.5 |
| Flavor component | 1 | 1 | 1 |

Compositions I, X and Y were evaluated in a home use consumer acceptability study. The results of the study demonstrate that an exemplary flavor component of the present invention provided improved consumer acceptability over similarly formulated compositions using flavor components different from those described herein.

## Claims

1. A flavor component for use in an oral care composition containing a metal salt, comprising:
from 30 to 50%, by weight, L-menthol;
from 5 to 10%, by weight, N-ethyl-para-menthan-3-carboxamide;
from 5 to 14%, by weight, anethole;
from 0.1 to 1%, by weight, cinnamic aldehyde;
from 0.1 to 1%, by weight, eugenol;
from 1 to 5%, by weight, eucalyptol;
from 20 to 40%, by weight, peppermint oil;
from 1 to 5%, by weight, spearmint oil; and
from 5 to 10%, by weight, corn mint oil.

2. The flavor component of claim 1, comprising:
from 35 to 45%, by weight, L-menthol;
from 5 to 8%, by weight, N-ethyl-para-menthan-3-carboxamide;
from 8 to 12%, by weight, anethole;
from 0.5 to 0.9%, by weight, cinnamic aldehyde;
from 0.5 to 0.9%, by weight, eugenol;
from 2 to 4%, by weight, eucalyptol; and
from 22 to 27%, by weight, peppermint oil.

3. An oral care composition comprising:
a) the flavor component of claim 1 or claim 2;
b) one or more metal salts selected from a zinc salt, a calcium salt, a copper salt, an iron salt, a magnesium salt, a manganese salt, and a combination of two or more thereof; and
c) an orally acceptable carrier;
wherein the total concentration of said one or more metal salts is from 0.01 to 5%, by weight of the composition.

4. The composition of claim 3, wherein at least one of said one or more metal salts is a zinc salt selected from: zinc oxide; zinc sulfate; zinc chloride; zinc citrate; zinc lactate; zinc gluconate; zinc malate; zinc tartrate; zinc carbonate; zinc phosphate; and a combination of two or more thereof.

5. The composition of claim 3, wherein the zinc salt is selected from: zinc oxide; zinc citrate; zinc gluconate; zinc glycinate; zinc lactate; and a combination of two or more thereof.

6. The composition of any one of claims 3 to 5, wherein the zinc salt is selected from: zinc oxide; zinc citrate; and a combination of two or more thereof.

7. The composition of any one of claims 3 to 6, wherein the zinc salt is zinc citrate.

8. The composition of any one of claims 3 to 7, wherein the zinc salt is a combination of zinc oxide and zinc citrate.

9. The composition of any one of claims 3 to 8, wherein the metal salt is present at a concentration of from 0.1 to 4%, by weight.

10. The composition of claim 9, wherein the metal salt is present at a concentration of from 1 to 3%, by weight.

11. The composition of claim 10, wherein the metal salt is present at a concentration of about 2%, by weight.

12. The composition of any one of claims 3 to 11, wherein the flavor component is present at a concentration of from 0.5 to 2.5%, by weight.

13. The composition of claim 12, wherein the flavor component is present at a concentration of from 0.8 to 1.5%, by weight; optionally about 1%, by weight.

14. The composition of any one of claims 3 to 13, further comprising one or more components selected from: a fluoride ion source; a tartar control agent; a buffering agent; an abrasive; and a combination of two or more thereof.

15. The composition according to claim 14, wherein at least one of said one or more components is a fluoride ion source selected from: stannous fluoride, sodium fluoride, potassium fluoride, sodium monofluorophosphate, sodium fluorosilicate, ammonium fluorosilicate, amine fluoride, ammonium fluoride, and a combination of two or more thereof.

## Patentansprüche

1. Geschmacksbestandteil zur Verwendung in einer Mundpflegezusammensetzung enthaltend ein Metallsalz, umfassend:
von 30 bis 50 Gew.% L-Menthol;
von 5 bis 10 Gew.% N-Ethyl-para-menthan-3-carboxamid;
von 5 bis 14 Gew.% Anethol;
von 0.1 bis 1 Gew.% Zimtsäurealdehyd;
von 0.1 bis 1 Gew.% Eugenol;
von 1 bis 5 Gew.% Eucalyptol;
von 20 bis 40 Gew.% Pfefferminzöl;
von 1 bis 5 Gew.% Speerminzöl; und
von 5 bis 10 Gew.% Ackerminzeöl.

2. Geschmacksbestandteil nach Anspruch 1, umfassend:
von 35 bis 45 Gew.% L-Menthol;
von 5 bis 8 Gew.% N-Ethyl-para-menthan-3-carboxamid;
von 8 bis 12 Gew.% Anethol;
von 0.5 bis 0.9 Gew.% Zimtsäurealdehyd;
von 0.5 bis 0.9 Gew.% Eugenol;
von 2 bis 4 Gew.% Eucalyptol; und
von 22 bis 27 Gew.% Pfefferminzöl.

3. Mundpflegezusammensetzung umfassend:
a) den Geschmacksbestandteil nach Anspruch 1 oder Anspruch 2;
b) ein oder mehrere Metallsalze ausgewählt aus einem Zinksalz, einem Kalziumsalz, einem Kupfersalz, einem Eisensalz, einem Magnesiumsalz, einem Mangansalz, und einer Kombination aus zwei oder mehreren davon; und
c) einen oral annehmbaren Träger;
worin die Gesamtkonzentration des einen oder der mehreren Metallsalze von 0.01 bis 5 Gew.% der Zusammensetzung ist.

4. Zusammensetzung nach Anspruch 3, worin das mindestens eine oder die mehreren Metallsalze ein Zinksalz ist ausgewählt aus: Zinkoxid, Zinksulfat, Zinkchlorid, Zinkzitrat, Zinklaktat, Zinkgluconat, Zinkmalat, Zinktartrat, Zinkkarbonat, Zinkphosphat und einer Kombination aus zwei oder mehreren davon.

5. Zusammensetzung nach Anspruch 3, worin das Zinksalz ausgewählt ist aus: Zinkoxid, Zinkzitrat, Zinkgluconat, Zinkglyzinat, Zinklaktat und einer Kombination aus zwei oder mehreren davon.

6. Zusammensetzungen nach irgendeinem der Ansprüche 3 bis 5, worin das Zinksalz ausgewählt ist aus: Zinkoxid, Zinkzitrat und einer Kombination aus zwei oder mehreren davon.

7. Zusammensetzung nach irgendeinem der Ansprüche 3 bis 6, worin das Zinksalz Zinkzitrat ist.

8. Zusammensetzung nach irgendeinem der vorhergehenden Ansprüche 3 bis 7, worin das Zinksalz eine Kombination aus Zinkoxid und Zinkzitrat ist.

9. Zusammensetzung nach irgendeinem der Ansprüche 3 bis 8, worin das Metallsalz bei einer Konzentration von 0.1 bis 4 Gew.% vorliegt.

10. Zusammensetzung nach Anspruch 9, worin das Metallsalz bei einer Konzentration von 1 bis 3 Gew.% vorliegt.

11. Zusammensetzung nach Anspruch 11, worin das Metallsalz bei einer Konzentration von etwa 2 Gew.% vorliegt.

12. Zusammensetzung nach irgendeinem der Ansprüche 3 bis 11, worin der Geschmacksbestandteil bei einer Konzentration von 0.5 bis 2.5 Gew.% vorliegt.

13. Zusammensetzung nach Anspruch 12, worin der Geschmacksbestandteil bei einer Konzentration von 0.8 bis 1.5 Gew.%, gegebenenfalls etwa 1 Gew.%, vorliegt.

14. Zusammensetzung nach irgendeinem der Ansprüche 3 bis 13, weiterhin umfassend ein oder mehrere Bestandteile ausgewählt aus einer Fluoridionenquelle, einem Zahnsteinkontrollmittel, einem Pufferungsmittel, einem Abrasivstoff, und einer Kombination aus zwei oder mehreren davon.

15. Zusammensetzung nach Anspruch 14, worin mindestens eines des oder der besagten Bestandteile eine Fluoridionenquelle ist, ausgewählt aus: Zinnfluorid, Natriumfluorid, Kaliumfluorid, Natriummonofluorphosphat, Natriumfluorsilicat, Ammoniumfluorsilicat, Aminfluorid, Ammoniumfluorid, und einer Kombination aus zwei oder mehreren davon.

## Revendications

1. Composant d'arôme pour une utilisation dans une composition de soin buccal contenant un sel métallique, comprenant :
de 30 à 50 %, en poids, de L-menthol ;
de 5 à 10 %, en poids, de N-éthyl-para-menthan-3-carboxamide ;
de 5 à 14 %, en poids, d'anéthole ;
de 0,1 à 1 %, en poids, d'aldéhyde cinnamique ;
de 0,1 à 1 %, en poids, d'eugénol ;
de 1 à 5 %, en poids, d'eucalyptol ;
de 20 à 40 %, en poids, d'essence de menthe poivrée ;
de 1 à 5 %, en poids, d'essence de menthe verte ; et
de 5 à 10 %, en poids, d'essence de menthe des champs.

2. Composant d'arôme selon la revendication 1, comprenant :
de 35 à 45 %, en poids, de L-menthol ;
de 5 à 8 %, en poids, de N-éthyl-para-menthan-3-carboxamide ;
de 8 à 12 %, en poids, d'anéthole ;
de 0,5 à 0,9 %, en poids, d'aldéhyde cinnamique ;
de 0,5 à 0,9 %, en poids, d'eugénol ;
de 2 à 4 %, en poids, d'eucalyptol ; et
de 22 à 27 %, en poids, d'essence de menthe poivrée.

3. Composition de soin buccal comprenant :
a) e composant d'arôme selon la revendication 1 ou la revendication 2 ;
b) un ou plusieurs sels métalliques choisis parmi un sel de zinc, un sel de calcium, un sel de cuivre, un sel de fer, un sel de magnésium, un sel de manganèse et une combinaison de deux ou plus de ceux-ci ; et
c) un support acceptable par voie orale ;
dans laquelle la concentration totale dudit ou desdits sels métalliques est de 0,01 à 5 %, en poids de la composition.

4. Composition selon la revendication 3, dans laquelle au moins l'un dudit ou desdits sels métalliques est un sel de zinc choisi parmi : l'oxyde de zinc ; le sulfate de zinc ; le chlorure de zinc ; le citrate de zinc ; le lactate de zinc ; le gluconate de zinc ; le malate de zinc ; le tartrate de zinc ; le carbonate de zinc ; le phosphate de zinc ; et une combinaison de deux ou plus de ceux-ci.

5. Composition selon la revendication 3, dans laquelle le sel de zinc est choisi parmi : l'oxyde de zinc ; le citrate de zinc ; le gluconate de zinc ; le glycinate de zinc ; le lactate de zinc ; et une combinaison de deux ou plus de ceux-ci.

6. Composition selon l'une quelconque des revendications 3 à 5, dans laquelle le sel de zinc est choisi parmi : l'oxyde de zinc ; le citrate de zinc ; et une combinaison de deux ou plus de ceux-ci.

7. Composition selon l'une quelconque des revendications 3 à 6, dans laquelle le sel de zinc est le citrate de zinc.

8. Composition selon l'une quelconque des revendications 3 à 7, dans laquelle le sel de zinc est une combinaison d'oxyde de zinc et de citrate de zinc.

9. Composition selon l'une quelconque des revendications 3 à 8, dans laquelle le sel métallique est présent à une concentration de 0,1 à 4 %, en poids.

10. Composition selon la revendication 9, dans laquelle le sel métallique est présent à une concentration de 1 à 3 %, en poids.

11. Composition selon la revendication 10, dans laquelle le sel métallique est présent à une concentration d'environ 2 %, en poids.

12. Composition selon l'une quelconque des revendications 3 à 11, dans laquelle le composant d'arôme est présent à une concentration de 0,5 à 2,5 %, en poids.

13. Composition selon la revendication 12, dans laquelle le composant d'arôme est présent à une concentration de 0,8 à 1,5 %, en poids ; éventuellement d'environ 1 %, en poids.

14. Composition selon l'une quelconque des revendications 3 à 13, comprenant en outre un ou plusieurs composants choisis parmi : une source d'ions fluorure ; un agent de lutte contre le tartre ; un agent tampon ; un abrasif ; et une combinaison de deux ou plus de ceux-ci.

15. Composition selon la revendication 14, dans laquelle au moins l'un dudit ou desdits composants est une source d'ions fluorure choisie parmi : le fluorure stanneux, le fluorure de sodium, le fluorure de potassium, le monofluorophosphate de sodium, le fluorosilicate de sodium, le fluorosilicate d'ammonium, le fluorure d'amine, le fluorure d'ammonium et une combinaison de deux ou plus de ceux-ci.
